# EUROPEAN PATENT APPLICATION

(11) **EP 1 602 365 A1**
(43) Date of publication of application: **07.12.2005**
(21) Application number: 04005008.0
(22) Date of filing: 03.03.2004
(51) Int. Cl.: A61K 9/70, B01J 13/00, A61L 15/44

(54) **Pharmaceutical composition for topical use in form of xerogels or films and methods for production**

(71) Applicant: Switch Biotech Aktiengesellschaft, 82061 Neuried (DE); Ludwig-Maximilians-Universität München, 80359 München (DE)
(72) Inventor: Stabenau, Anke, 81477 München (DE); Winter, Gerhard, 82377 Penzberg (DE); Schmidt, Roland, 91801 Markt Berlzheim (DE)
(74) Representative: Zwicker, Jörk

(57) **Abstract**

The present invention relates to dry delivery system comprising a xerogel or film with applied active ingredients for topical active ingredient delivery or other purposes. Said delivery system are obtainable by a method according to the invention. The present invention also provides for methods for achieving defined localization of stable or unstable active substances on dry xerogels or films, which can be reconstituted into hydrogels. From the obtained delivery systems, the active substances are released with advantageous release kinetics.

## Description

### Field of the invention

This invention relates to a dry active ingredient delivery system for nasal, ocular or dermal use or other therapeutic or diagnostic applications and methods for preparing them. More particularly it relates to a xerogel or film, onto which therapeutically active substances are applied as small droplets and may be dried in vacuum. The therapeutic substances can be applied in defined patterns on one or more surfaces of the xerogel or film. Such a system can be used as a storage stable and dry active ingredient delivery system for pharmaceutically and/or biologically active ingredients in the field of cosmetics and medicine. Before use or throughout application in a moist environment (e.g. a wound) the system is rehydrated, thus serving as a hydrogel loaded with therapeutic substances, which are released at a controlled rate. Such a system can be used for moist wound healing, for nasal, ocular or dermal delivery of therapeutic substances or for other purposes.

### Background of the invention

The present invention can for example be used for nasal, ocular or dermal delivery of therapeutic substances. It is especially useful for moist wound healing. The growing number of patients with diabetes mellitus, venous insufficiency and other chronic diseases and injuries has resulted in an increased incidence of chronic non healing soft-tissue wounds ({2200}). Apart from causing great costs to the public health system, chronic wounds give raise to a very painful, distressing condition of the patient and may even lead to amputation. Therefore adequate treatment and promotion of dermal wound healing is necessary.

The mechanisms of wound healing in general and the characteristics of different wound healing phases are well known. Since 1962 moist wound healing has become a widely accepted treatment ({2202}). Already many different moist bandages, like hydrocolloid, hydropolymer or alginate systems are on the market. This bandages shall ensure a moist environment in the wound. Sometimes they additionally take up wound secretions while they swell or in exchange with solutions, which are incorporated into the bandages (for example ringer solution). All these wound dressings consist of a swollen or swellable polymer and sometimes of a water resistant backing layer, but they usually do not contain any therapeutic active substances.

Next to a moist environment, adequate concentrations of growth factors are necessary to promote healing. In the 1970's the inductive effect of platelet derived factors and other cytokines was first described ({2201}) and since then many studies have proved the clinical usefulness of these factors ({2200}). The necessary balance and concentration of different growth factors for the promotion of healing is often disturbed, especially with elderly people and patients with diabetes or autoimmune disorders ({2201}). Therefore it has proven to be useful to apply wound healing factors, like PDGF, TGF-β, F XIII, KGF-2 or EGF to enumerate just few, topically into wounds.

However growth factors and also enzymes, which promote wound healing in the first cleaning phase, are proteins and therefore quite unstable and sensitive molecules. When stored in aqueous solutions at room temperature many proteins do not remain stable, they may aggregate and lose activity rapidly. EGF in an aqueous formulation for example lost 40% of its activity within two weeks ({2203}). To achieve longer storage stabilities protein solutions have to be stored under defined conditions at deep temperatures (-20°C or 4-8°C). Therefore these aqueous products have technical, economical or handling drawbacks. E.g. Regranex®, a gel with rh (recombinant human) PDGF-BB, has to be stored in a refrigerator. Also the solution Eurokinin, a cleaned solution of the patient's own growth factors, must be stored in a freezer. This leads to additional problems as it has to be carefully thawed before application, which decreases patient and medical personal compliance.

An alternative to deep temperature storage is the stabilisation of a sensitive substance in dry products. It is a widespread method in the pharmaceutical technology to embed sensitive substances like proteins in dry, amorphous matrixes to ensure low aggregation rates. Additionally chemical degradation reactions are decreased and thermal stability is increased in a dry environment. Thus there is a high need for the development of dry, storage stable active ingredient products. These dry products have to be reconstituted before or during use, as for wound healing the application of moist products, like solutions or preferably hydrogels, is necessary. In Varidase® N Gel the active compound, a enzyme, is separated from the gel and brought to market as a dry powder. Before application the powder has to be dissolved in water and added to the gel, which then shows only a short shelf live. This preparation step is time consuming and may lead to problems in reproducibility or dosage. It would be preferable to have both, the sensitive active substance and the gel, present in one single system, which exhibits a dry storage form. Thus xerogels or films with incorporated active substances are a promising tool to ensure stability in the dried form and to overcome deep temperature storage. Such systems allow the combination of active substance and matrix in a single ready-to- use system.

"Xerogel" according to the present invention is to be understood as porous, sponge-like matrix obtainable from a hydrogel e.g. by freeze-drying comprising at least one gelating substance wherein the matrix has the potential to swell and form hydrogels when in contact with aqueous solutions.

"Film" according to the present invention is to be understood as polymer-based foil of flat-shaped form of uniform thickness and consistency obtainable from a hydrogel by drying, e.g. evaporative drying or by casting from organic solutions. The matrix has the potential to swell and form hydrogels when in contact with aqueous solutions

"Dry" according to the present invention is to be understood as containing a very low content of water, preferably less than 5% (w/w) moisture, more preferably less than 2% (w/w) moisture, especially preferably less than 1% (w/w) moisture. Moisture can be determined by coulometric Karl-Fischer titration, for example using KF 373 (Metrohm Gmbh&Co, Filderstadt, Germany).

"Microdroplet" according to the present invention is to be understood as droplet which, when applied on a film or xerogel does not substantially change the shape of said film or xerogel. Preferably, a microdroplet does not have a volume bigger than 10 µl, more preferably not bigger than 200 nl.

"Carrier" is to be understood as a composition useful for delivery of active substances for the medical treatment or prevention of diseases and /or disorders or for cosmetic treatment of conditions of the body.

"Active ingredient" is to be understood as any substance which causes a biological effect, either directly or when released from its pro-drug form in vivo and which is thus beneficial for the medical treatment or prevention of diseases and /or disorders or for cosmetic treatment of conditions of the body.

"Surface" of a dry hydrogel or film carrier is to be understood as any surface of the carrier which is confined by edges; thus in case the carrier has a spherical shape, only one surface exists, whereas in case the carrier has approximately cube shape, the carrier has 6 surfaces, and in case the carrier has approximately cylindrical shape, it has 3 surfaces.

"Essentially no change in shape" is to be understood as that after the appliance of the droplets there is no substantial swelling or shrinking of the carrier as a whole, i.e. there is no substantial increase or decrease in volumeof said carrier itself.

"surface areas" of a dry hydrogel or film carrier is to be understood as any area being part of or, at maximum, being equivalent to a surface.

Sometimes it may be necessary to have two or more active ingredients in one system, as the combination of multiple therapeutic active substances often shows synergistic effects. Even though these active ingredients do not interact in a negative manner in the patient's body it might be necessary to separate them throughout storage, as different active ingredients often need very different stabilising environments (pH, salts, excipients, etc.). To avoid separate products or additional powders for each active ingredient, which would have to be combined before use, the production of one single, ready-to-use system, in which the active ingredients are separated throughout storage, and if desirable, also during delivery in the body, would be very useful.

Defined geometric active ingredient patterns on a matrix would additionally allow a locally defined application of substances, which can not be guaranteed by solutions or hydrogels.

A dry storage system can either be rehydrated before use with pure water or throughout use with aqueous body fluids, for example exudates within the wound. Thereby the xerogel or film takes up water, swells and forms a hydrogel. Hydrogels are preferred over solutions with low viscosity as they keep the wound moist, do not evaporate fast and therefore have to be applied only once daily. The solution Eurokinin for example has to be applied continuously onto a compress on the wound. Regranex® is a hydrogel, which shows good wound healing and handling properties, but bad storage stabilities. A desirable, optimal active ingredient product would have a dry, storage stable form and could be rehydrated to a hydrogel before or during use. Such storage stable forms need not be stored at very low temperatures and thus allow also easy and cheap transportation. Moreover, such products may also be stored by the patient itself without complications. Thereby high costs which occur when treatment has to be effected at hospitals are avoided.

After rehydration the applied active ingredient substances should dissolve rapidly leading to a fast active ingredient release or, as a second option, they could be released in a controlled slow release manner. It is especially beneficial to achieve a locally high concentration. As highly active ingredients, like e.g. proteins, are very expensive, it is of high interest not to waste any active ingredient. None of the existing formulations fulfils all these criteria. If the active ingredient is homogenously dispersed in a gel matrix it must be accepted, that only a minor part of the active ingredient will be absorbed by the target tissue from the contact surface area between formulation and tissue and that a bulk amount will be lost within the gel by adsorption to occlusive patches or may be eroded, washed or swept away over time. On the other hand, a rather high initial concentration of the active ingredient in the formulation is desirable to allow a significant concentration gradient to build up and to achieve the necessary driving force for diffusion of the active ingredient from vehicle into the target tissues. Therefore an optimum has to be found between limiting the expensive active ingredient losses and the minimum overall concentration necessary.

Additionally other problems of existing topical products have to be faced as for example the absence of exact and reproducible dosage. All hydrogels on the market (like Regranex®) are dosed by the amount of the applied hydrogel strand. This is not very exact and not reproducible. It would be useful to have a dry, ready-to-use, single dose product covering a defined contact area, which could also be cut reproducible into pieces to achieve defined doses.

For fresh wounds, ocular mucosa etc. all applied materials must be sterile. This is not the case with some of the existing, protein containing xerogel-, film- or hydrogel-products. These non-sterile products present quite a risk and are not acceptable for many applications. Regranex® for example is a non-sterile hydrogel, which can only be used with non-infected wounds. Gels with incorporated sensitive active ingredients, like proteins, can not be sterilised easily. Many hydrogels could be sterilized by moist heat or radiation, yet most sensitive active ingredients, which were incorporated into the gel, would loose stability and activity throughout these processes. Sterile active ingredient solutions can be prepared by filtration through a 0,22 µm filter unit. Therefore both parts of the medicament - gel and active ingredient - may be sterilised separately and could be combined afterwards. Additionally it would be advantageous not only to sterilise, but also to dry the hydrogel separately. Adequate drying conditions for a active ingredient-free hydrogel can be determined much easier, when only the physical properties of the xerogel or film and not the stability of the incorporated sensitive active ingredient have to be borne in mind. Therefore it would be desirable to prepare and sterilise a active ingredient-free xerogel or film first and have a method to add a sterile active ingredient solution afterwards under aseptic conditions without rehydrating the xerogel or film. Altogether a sterile active ingredient product with stable and active ingredients would be guaranteed.

In general the preparation of a active ingredient containing xerogel or film may cause many problems. First of all, in order to prepare a homogeneous formulation, the active ingredients have to be mixed with the swellable polymers in water. This often causes shear stress for the active ingredients. Additionally, hydration and swelling of the hydrogel takes quite a time, at least some hours, mostly one day. Throughout this time the incorporated sensitive active ingredients are in an aqueous environment normally at room temperature, which often destabilises sensitive active ingredients. To stabilise sensitive active ingredients in an aqueous gel medium and throughout drying, additives are necessary, which often have a negative influence on the swelling or rehydration behaviour of the gel. Therefore the formulation is always a compromise between active ingredient stability and optimal gel or film formation. It is desirable to have two separate formulations for the xerogel/film and the protein.

Overall it would be a great advantage to have a active ingredient delivery system based on hydrogel, which
(i) has a dry storage form, and
(ii) which can, if desired, be produced as a sterile product without heat stress for the active ingredients, and
(iii) which offers the opportunity to apply active ingredient solutions onto a xerogel or film without rehydration of the xerogel or film, thereby limiting the stress to the material used and
(iv) which offers the opportunity to separate multiple active ingredients on one single xerogel or film, even if they are usually regarded as being non-compatible and
(v) which exhibits a defined pattern of applied active ingredients, which allows the application of a defined active ingredient dose and
(vi) which forms a hydrogel after rehydration and achieves appropriate release kinetics and, at the same time, a high concentration of active ingredient at the release side.

Such a system is of interest especially for nasal, ocular or dermal or transdermal active ingredient delivery or for other purposes, whenever defined release kinetics for active ingredients are desired like for implants for therapeutic purposes. Such system is also of interest as intermediate product for the manufacture of a delivery system suitable for medical or cosmetic purposes; e.g. a delivery system according to the invention may be punched into tiny pieces which are then suspended in a liquid. Such a suspension can then filled in an applicator suitable for mucosal or nasal delivery.

Some of these desired properties have already been realized in known products, but none of these products does fulfil all requirements and some disadvantages remain.

To overcome the low storage stability of aqueous solutions or hydro gels, lyophilised products of many different formulations are claimed for example in EP 0 127 597 and US 5,189,148. These formulations may contain gelling agents and therefore form xerogels, yet a controlled swelling behaviour to a hydrogel upon rehydration or a controlled active ingredient release kinetic is not described.

In EP 0 308 238 Al a lyophilised xerogel ("...formulation with water soluble polymers for imparting viscosity...") with a human polypeptide growth factor is claimed. This product enables moist wound healing, forms a hydrogel of controlled viscosity and shows a sustained release. Yet nothing is said about it's sterility and as with all gels it can not enable a fast and initially high active ingredient release. As the active ingredient is incorporated into a gel, not all active ingredients or excipients can be used because of possible incompatibilities between active ingredient and excipient. Additionally no pattern and no separation of two or more active ingredients on one xerogel is possible according to that invention.

Apart from it's sterility the xerogel described in US 5,192,743 is similar to the one mentioned above and shows the same disadvantages. Sterility can only be gained by separately sterilising the protein and the gel-ingredients, which afterwards have to be stored for 24 hours at 5°C to allow the cellulose to hydrate. Finally both product parts are mixed and lyophilised. This is quite inconvenient and the long hydration time of the sterilised gel enlarges the risk of recontamination.

A compressed xerogel, which is especially soft and flexible and can conveniently be placed directly into the wound is described in EP 0 533 820 B1. The dry and storage stable product enables a defined dose and can additionally be cut or punched into pieces. Disadvantageous is that two or more incompatible substances can not be combined in the system and that no defined active ingredient patterns are formed. Like all other xerogels with incorporated active ingredients this product is limited in the choice of excipients, as all excipients have to be capable of forming xerogels of suitable physical properties and at the same time shall not influence the incorporated active ingredient in an negative manner. If two different formulations for the xerogel and the protein solution could be used this would be advantageous. A high initial release form the rehydrated gel is desired and even though the patent claims such a "fast release of some medicaments" no exact data is given on the diffusion rate from the rehydrated hydrogel.

An alternatively fast or sustained release is gained by the collagenase containing film described in DE 195 03 38 (also US 6,117,437) by the choice of the film forming polymer (water soluble or swellable, non water soluble). The release kinetics depend on the dissolution or erosion time of the matrix, which might take some time due to the small volumes of aqueous secretions available in a wound. The dissolution time could be fastened by rehydration of the dry matrix outside the wound with larger volumes. Active ingredients that are not incorporated in a gel, but applied on top of a xerogel or film could be dissolved and released very fast as they are independent from the dissolution or erosion time of the gel.

For collagenase containing medicaments it is especially important to avoid any application of collagenase outside the wound on healthy skin. In patent DE 195 03 38 this shall be guaranteed by cutting the product into pieces smaller than the wound. An additional impermeable backing layer, which is laminated onto the product, shall regulate the active ingredient release into a specific direction. It would be better to have a defined small, but highly concentrated active ingredient pattern in the middle of a product and a active ingredient-free edging around. If the pattern is rapidly soluble and the rest of the product is not, this would simultaneously regulate the release direction.

In the examples of the patent DE 195 03 38 the drug containing films are dried by convective drying at 45°C. Not all sensitive active ingredients stay stable at this high temperature and some might also be destabilised by the film formers itself.

The preparation of a hydrogel, which might be dried later on, sometimes takes quite long. Tae-Wan Lee, Jin-Chul Kom and Sung-Joo Hwang ({2230}) describe the preparation of a Triclosan containing acne hydrogel patch by crosslinking. To form semi-solid gels this process took at least 12 hours at 25°C, 40°C or 50°C. Especially sensitive active ingredients, like proteins will not survive this temperatures in an aqueous environment without loss of activity. Hydration and swelling of hydrogels normally even takes about one day.

In the light of the existing prior art to resolve the problem of a optimal, topical dosage form also suitable for unstable active ingredients for treatment of wounds, mucosa or other tissues, a solution can be proposed by combining already existing approaches in a innovative and inventive way: Based on the concept of dry hydrogels (i.e. xerogels or films) small amounts of highly concentrated active ingredient solution could be applied in predetermined geometric patterns preferably on one side of such hydrogels. Thereby advantageous release kinetics could be achieved due to the local high concentration of the active ingredient on at least one side of such hydrogels, which then should be brought into close contact with the tissue to be treated. By such means it is achieved, that the total loss of active ingredient is minimized; moreover the spatial separation of two or more active ingredients can be realized if desired, the production of sterile samples is guaranteed if desired and dry, storage stable products are formed. Application of the solution can be carried out for example by means of robotic spotters as used for analytical microarray production in the cause of the development of high throughput analytical systems which are well known to a person skilled in the art. Such spotters are e.g. known from US 6,642,054 and references therein. In a preferred embodiment of the invention described below, the microdroplets are applied on one or more surfaces or surface areas of the carrier by means of printing or spotting, preferably by piezoelectric printers, more preferably by printers, which use a syringe pump and a high-speed micro-solenoid valve. For application of a liquid containing active substances, also other means can be used, known to the person skilled in the art for example spraying or stamp transfer or the like.

### Summary of the invention

The present invention relates to dry delivery system comprising a xerogel or film with applied active ingredients for topical active ingredient delivery or other purposes. Said delivery system are obtainable by a method according to the invention. The present invention also provides for methods for achieving defined localization of stable or unstable active substances on dry xerogels or films, which can be reconstituted into hydrogels. From the obtained delivery systems, the active substances are released with advantageous release kinetics.

In accordance with the invention, one ore more defined doses of one ore more concentrated active ingredient formulations are divided into microdroplets, which are printed in patterns on a xerogel or film. Preferably, such pattern is regular in order to obtain reproducible release kinetics and a defined dosing. When the delivery system, i.e. the xerogel or film with applied active ingredient(s) comes into contact with fluids (water or aqueous solutions or body fluids throughout application) it is rehydrated and forms a hydrogel, whereby the applied active ingredients are dissolved and released at a controlled rate from the hydrogel leading to a locally high concentration.

### Detailed description of the Invention

It has been found that in the presence of moisture some active ingredients, for example hGH or EGF, aggregate and loose biological activity during storage. Therefore it is impractical to store aqueous preparations containing such active ingredients. This invention provides means for preventing loss of activity by providing stable dry delivery systems containing the active ingredient. The delivery systems of the present invention are therefore especially useful for active ingredients which are selected from protein, peptide, RNA, DNA or any other substance potentially unstable in a formulation especially for proteins and peptides. Thus, in addition to the advantageous release kinetics suitable for all active substances, the delivery system of the invention in addition thus offers additional advantages for unstable active substances.

A dry xerogel or film matrix for example can be obtained from a hydrogel by a freeze-drying or convective-drying method according to processes known to a person skilled in the art. In a preferred embodiment, the dry xerogel carrier is formed from a hydrogel by freeze-drying processes. In another embodiment, the dry film carrier is formed from a hydrogel by evaporative-drying processes, preferably air-drying, vacuum-drying or convective-drying. Also, the dry film carrier may be obtained by extrusion or casting from organic solvents.

In an embodiment of the present invention, the dry xerogel or film carrier contains one or more swellable, dissolvable or erodable polymers. The base material, i.e. said polymers, present in the xerogel may be appropriate gelling agents acceptable for dermal, ocular, nasal or other desired use. Gelling agents or gel-forming agents, which possess adequate mechanical, physiological and release properties, are preferably polysaccharides, like alginates, pectins, carrageenans or xanthan, starch and starch derivatives, gums like tragacanth or xanthan gum, collagen, gelatin, galactomannan and galactomannan derivatives, chitosan and chitosan derivatives, glycoproteins, proteoglycans, glucosaminoglycans, polyvinyl alcohol, polyvinylpyrrolidone, vinylpyrrolidone/vinyl acetate copolymers, high molecular weight polyethylene glycols and/or high molecular weight polypropylene glycols, polyoxyethylene/polyoxypropylene copolymers, polyvinyl alcohol, polyacrylates and/or polymethacrylates, polylactides, polyglycolides and polyaminoacids and cellulose derivatives. Especially preferred gel-forming agents are selected from cellulose derivatives, especially cellulose ether compounds, like methylcellulose, hydroxypropylcellulose, hydroxyethylcellulose, hydroxypropylmethylcellulose, sodium carboxymethylcellulose, ethylcellulose, cellulose acetate phthalate, hydroxypropylmethylcellulose phthalate, cellulose acetate succinate and ethylcellulose succinate.

The carrier may contain, if desired, one or more additional excipients like sugars, sugar alcohols, surfactants, amino acids, antioxidants, polyethylene glycols

The shape of a dry hydrogel or film carrier useable according to the present invention may differ and are usually not critical to the invention. However, application of a regular pattern of microdroplets with active substances and also obtaining homogeneous release kinetics will often be easier to achieve with approximately even surfaces which are present in cubes, cuboids sheets or cyliders. For example such carrier may be approximately spherical, hemispherical, cubic, cuboid, a sheet, a regular or irregular polyhedron or may be irregularly shaped.

In a preferred embodiment, carrier has at least two surfaces separated by edges. In a more preferred embodiment the carrier has the form of a cylinder, a parallelepiped, a cube or a cuboid.

In a preferred embodiment, the microdroplets are applied to one or more surfaces, preferably one or two surfaces, where applicable; i.e. if more than one surface exists. In a preferred embodiment, the microdroplets are applied in a way that the carrier essentially does not change its shape.

The problem to be solved is the combination of a rather hydrophilic active ingredient dispersed or dissolved in a liquid, preferably in an aqueous solution, which is, in an especially preferred embodiment a stabilizing formulation, with pre-dried hydrogel or film matrices suitable for delivery of active ingredients without disturbing the quality of such matrices. Surprisingly it has been found that by means of dispensing and printing steps carried out according to the invention it is possible to apply significant active ingredient quantities onto one or more surfaces or surface areas of a dry xerogel or film matrix in a controlled manner allowing a very accurate definition of the dose and any geometrically defined pattern without dissolving, shrinking or otherwise disturbing the quality, volume and moisture of the matrix itself.

The method for producing a delivery system according is described as follows: The invention relates to a method of producing a delivery system for medical and/or cosmetic use comprising a carrier and at least one active ingredient characterized by following steps:
(i) preparing a liquid wherein at least one active ingredient is dissolved or dispersed
(ii) optionally sterilising the liquid
(iii) preparing and optionally sterilizing a dry xerogel or film carrier
(iv) applying microdroplets of the liquid according to steps (i) or, if applicable, (ii), to at least one surface area of a dry xerogel or film carrier obtained from step (iii)
(v) optionally repeating step (iv) at least one time, and
(vi) optionally repeating steps (i) to (v) with a liquid containing another active ingredient at least one time
(vii) optionally vacuum-drying or freeze-drying the system obtained by above steps.

In a preferred embodiment, the delivery system is vacuum-dried in step (vii).

It has to be understood that the sterilizing steps may in addition also be performed at the end, namely after manufacturing the delivery system according to above method, if desired.

Vacuum-drying or freeze-drying steps may be performed in addition after step (v) of the above-described method.

In a preferred embodiment, the liquid of above method is a solution or a dispersion, preferably a solution. In case when unstable active substances are used, the liquid is a stabilizing solution or stabilizing formulation.

If necessary the printed xerogel or film delivery systems can be dried in vacuum for a short time period to lower residual moisture (preferably below 5%, more preferable below 2%, especially preferably below 1%). Thus the present invention also relates to a method as described above characterized in that the system is dried in vacuum after applying microdroplets, i.e. after step (iv) of above-described method, to lower the residual moisture preferably below 5%, more preferably below 2%, especially preferably below 1% if necessary. In another embodiment of the present invention, the carrier, on which the microdroplets are applied, is heated preferably to not more than 40°C, more preferably to not more than 30°C for drying, that is, after step (iv) of above-described method.

More specifically, the invention relates a method of producing a delivery system for medical and/or cosmetic use comprising a carrier and at least one active ingredient characterized by following steps:
(i) preparing a liquid wherein at least one active ingredient is dissolved or dispersed
(ii) optionally sterilising the liquid
(iii) preparing and optionally sterilizing a dry xerogel or film carrier
(iv) applying microdroplets of the liquid according to steps (i) or, if applicable, (ii) to at least one surface area of a dry xerogel or film carrier obtained from step (iii)
(v) optionally repeating step (iv) at least one time, and
(vi) optionally repeating steps (i) to (v) with a liquid containing another active ingredient at least one time
(viii) vacuum-drying or freeze-drying the system obtained by above steps.

In an even more preferred embodiment, the invention relates to a method comprising following steps:
(i) preparing a liquid wherein at least one active ingredient is dissolved or dispersed
(ii) sterilising the liquid
(iii) preparing and sterilizing a dry xerogel or film carrier
(iv) applying microdroplets of the liquid according to step (ii) to at least one surface area of a dry xerogel or film carrier obtained from step (iii)
(v) optionally repeating step (iv) at least one time, and
(vi) optionally repeating steps (i) to (v) with a liquid containing another active ingredient at least one time
(vii) vacuum-drying or freeze-drying the system obtained by above steps.

This method is especially useful for the preparation of delivery systems for use in medicine where sterile products are usually required.

The carrier to be used may be a xerogel or film.

In another preferred embodiment the invention relates to a method comprising following steps:
(i) preparing a liquid wherein at least one active ingredient is dissolved or dispersed
(ii) optionally sterilising the liquid
(iii) preparing and optionally sterilizing a dry xerogel or film carrier
(iv) applying microdroplets of the liquid according to steps (i) or, if applicable, (ii), to at least one surface area of a dry xerogel or film carrier obtained from step (iii)
(v) optionally repeating step (iv) at least one time, and
(vi) repeating steps (i) to (v) with a liquid containing another active ingredient at least one time
(vii) optionally vacuum-drying or freeze-drying the system obtained by above steps.

In a preferred embodiment the microdroplets are applied in a defined, regular pattern. In another preferred embodiment, at least about 10, more preferably, at least about 100, especially preferably at least about 500 microdroplets are applied on a carrier.

In an embodiment of above-described methods of the invention, the microdroplets of step (iv) are placed separately or with contact to each other or on top of each other, preferably separately. More preferably, the microdroplets are applied in a way that defined spots containing at least one active ingredient are obtained.

In another embodiment of above-described methods of the invention, the microdroplets of step (vi), containing another active ingredient, are applied separately or with contact to or on top of the microdroplets of the first round of step (iv) (i.e. containing the first active substance), preferably separately from microdroplets of the first round of step (iv). In a more preferred embodiment, the microdroplets of step (vi), containing another active ingredient are applied to a different surface than the microdroplets applied in the first round of step (iv).

The invention also relates to a delivery system suitable for medical and/or cosmetic use comprising a carrier and at least one active ingredient, obtainable by a method described above.

The invention also relates to a delivery system for medical and/or cosmetic use comprising a dry xerogel or film carrier and a pattern of dried microdroplets, containing one or more active ingredients. In a preferred embodiment pattern is regular.

The present invention also relates to a method of rehydrating a delivery system for medical and/or cosmetic use comprising a carrier and at least one active ingredient, obtainable by a method described above characterized in that the composition is brought into contact with an aqueous solution or water outside the patient to be treated.

Also, the present invention relates to such delivery system which is rehydrated by such method. Also, the present invention relates to such rehydrated delivery system which is rehydrated by administration in or on the body, for example by placing in contact with wound fluid.

In a preferred embodiment, for such rehydrated delivery system, a fast release of at least one active ingredient is observed.

In another preferred embodiment, for such rehydrated delivery system a slow, controlled release of the active ingredient or ingredients is observed.

In another embodiment, the invention relates to a composition for cosmetic or medical application on skin or on skin wounds, comprising a delivery system or a rehydrated delivery system as described above and an inert support, preferably selected from adhesive strip, adhesive wrap, bandage, gauze bandage or compress system or others.

In order to stabilize some active ingredients, especially proteins, buffer agents, excipients against dehydration stress ("lyoprotectants") and non-ionic surface active components may be included into the liquid suitable for protein formulation. Thus, in a preferred embodiment, the liquid , of step (i) of above-described methods of the invention is an aqueous solution, especially preferably a stabilizing formulation and may contain one or more excipients selected especially from sugars, sugar alcohols, surfactants, amino acids, buffers, lyoprotectants or antioxidants.

Even temperature-sensitive active ingredients or active ingredients that do not stay stable, when mixed with the xerogel or film matrix materials in an aqueous medium, can be used. The presented invention causes no heat stress for the applied active ingredients. The contact time between the applied aqueous droplets and the dry xerogel or film is very short, as the applied droplets dry very fast due of their small size. Additionally the contact area is very small due to the small droplet size. The preparation process according to the invention allows to fulfil all needs due to physicochemical properties of the active ingredients used.

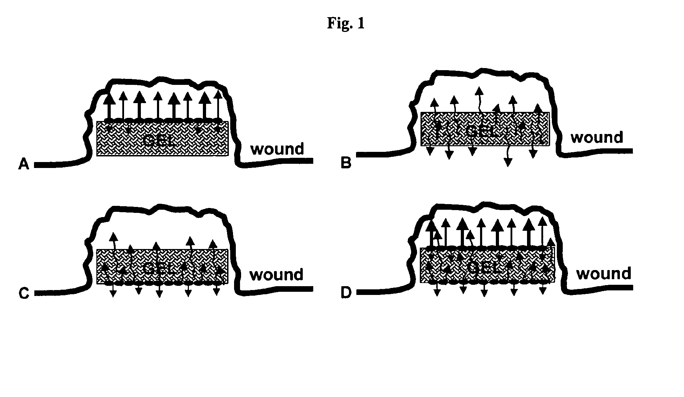

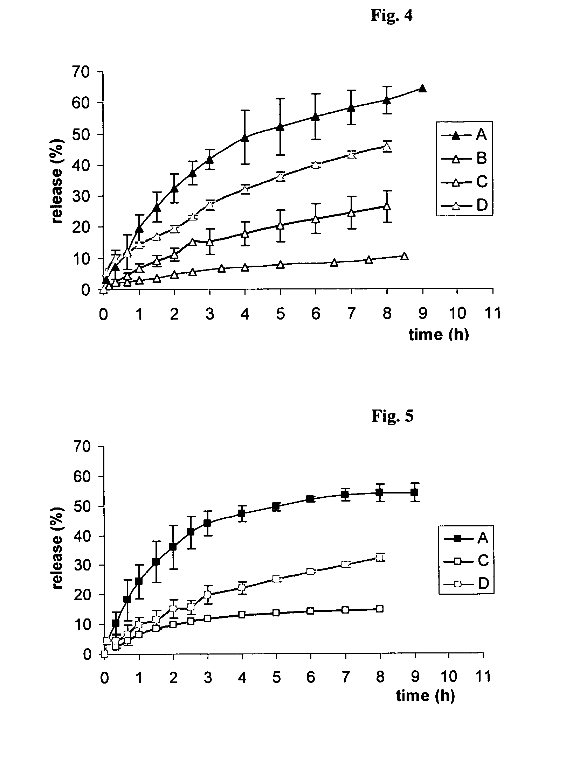

The presented invention allows the easy production of sterile products. Non-sterile products present a risk to the patient and are not acceptable for many applications. Gels with incorporated active ingredients can not be sterilised easily, as many active ingredients do not stay stable throughout heat or radiation sterilisation processes. Additionally it is often difficult to find an adequate drying process for gels with incorporated sensitive active ingredients. The presented method separates gel and active ingredient throughout these production steps. First an active ingredient-free, sterile xerogel or film is created by sterilisation of a hydrogel, for example by hot vapour or radiation and drying, especially freeze-drying. Afterwards a sterile active ingredient formulation, which had been produced by sterile filtration under aseptic conditions, is applied onto the sterile xerogels or films with the aid of aseptic production techniques. Thus, in an embodiment, the present invention relates to a method as described above wherein the sterile liquid, which is preferably a solution, according to step (ii) of said method containing at least one active ingredient is applied on a sterile carrier according to step (iii) of said method under aseptic conditions and with accordance to step (iv) of above methods, whereby a sterile delivery system is produced. This approach avoids sterilisation stress for the active ingredients and allows drying parameters, which are optimal for the xerogel or film, but which would harm the active ingredients. As the active ingredient solution is not incorporated into the gel, but printed onto the xerogel or film surface(s) or surface area(s), whereon it dries very fast, a minimal contact area and time is guaranteed. Incompatibilities between active ingredient and gel formulation, which are often enhanced throughout drying or sterilising, are minimized hereby. Similarly, the delivery systems of the present invention are especially suitable for combining two or more active agents which are incompatible. By the application of microdroplets containing one active substance on the carrier separately from the microdroplets containing other active substances, incompatibilities which might occur during storage, manufacture or release can be avoided. Moreover, such spatial separation may be designed according to the substances: for example such active substances may be applied to opposite sides of a carrier, resulting in temporally and/or spatially discrete release of both compounds (see e.g. Fig. 1D, Figure 4). In another embodiment, at least one active substance is incorporated in the carrier (see Fig. 1B, fig. 4) and at least one other active substance is applied according to the method of the invention. Also, in this case incompatibilities can be avoided due to the different release kinetics leading to spatial and temporal separation of the substances. The incompatible active substances or incompatible solutions comprising them may be applied by any of these methods or combinations thereof: due to the variably adjustable release kinetics depending on the site of application of the microdroplets, as well as the possibility the apply microdroplets separately or even on different areas, and the opportunity to also incorporate other active substances into the carrier provides versatile means for producing delivery systems for cosmetic and/or medical use which are optimally designed to fit to the properties of the compounds employed and to the desired release kinetics. In addition, also pro-drugs may be employed as active substances offering an additional opportunity to adapt the delivery systems to the medical or cosmetic needs by providing delayed activation in vivo and hence, spatial separation from another active substance which would in its active form be incompatible.. Additionally the presented method allows the use of active ingredient formulations, which would disturb and/or inhibit adequate drying, swelling or physical characteristics of the xerogel or film carrier. When they are applied, preferably printed on the dry xerogel or film they do not influence the gel characteristics, which would happen, if they would be incorporated into the gel.

An advantage of the presented invention is the adjustable droplet size. Dependent on the xerogel or film porosity, structure and dissolvability a droplet size can be chosen, which do not dissolve the xerogel or film or cause irregular swelling of the carrier. Usually a wide range of droplet sizes are usable. Therefore an adequate droplet volume can be determined accordingly to the available xerogel or film properties, namely whether they swell easily or not. Also the amount of droplets applied may differ, depending on the active ingredient dose and the concentration of the solution. However, the size of the microdroplets will usually be small in order to avoid rehydration, swelling and/or change in shape of the xerogel or film carrier. On the other hand, a sufficient amount of active ingredient has to be applied. Preferably, the microdroplets have a volume between about 0,05 nl and 10 µl, more preferably between about 0,5 nl and 200 nl, most preferably between about 10 nl and 100 nl.

Another important aspect of the presented invention is the possibility to define the applied patterns to the will of the user. Parts of the xerogel or film can be applied with active ingredients, whereas other parts can be left free. For example a product with a active ingredient free edging around the applied active ingredient pattern can be guaranteed. Thus, in a preferred embodiment, the microdroplets are applied in the inner area of a carrier surface, leaving an active ingredient-free edging around said surface area. If desired any contact between the applied active ingredient spots can be prevented. Additionally the active ingredients can be applied on more than one side of the xerogel or film. Therefore two or more incompatible formulations can be applied separated by open space on one single xerogel or film, without activity loss caused by the contact of incompatible substances. Also, in a preferred embodiment, the pattern in which the microdroplets are applied is regular, especially preferably the distance between microdroplets is constant in order to achieve homogeneous and reliable release kinetics.

It is possible to prepare compositions of the present invention, especially the printed xerogels or films shown in the examples, from which pieces of a desired size can be cut or punched out to adapt the desired dose and/or to the desired size, for example to fit a wound.

In a preferred embodiment, the compositions of the invention contain at least one active ingredient that promotes wound healing, preferably a wound healing factor, enzyme or proteinase inhibitor.

According to published data xerogels or films can be produced as soft flexible sheets having a porous and flexible structure, which can easily be placed directly on a wound. The application of active ingredients on a xerogel or film with the presented method does not change the structure of the xerogel or film. Therefore printed, soft xerogels or films can also be placed directly on a wound. Hence the patient does not have to make a reconstitution of theses products.

Dependent on the therapeutic aspect of the applied active ingredients the delivery system for cosmetic and/or medical use obtainable by the process of the invention can be used, for example for treating and/or preventing wound healing disorders, like diabetic, venous or neuropathic ulcers or infected wounds, ophthalmologic, nasal diseases or skin disorders, e.g. selected from psoriasis, dermatoses, eczema, urtikaria, lupus erythematous, vitiligo, pigmentation disorders or atopic dermatitis. It may also be used, depending on the active ingredient, whenever controlled or fast release is desired, for example for transdermal delivery of active ingredients or for implants. If enough body fluid, for example wound secret, nose secret or eye drops, is existent at the place of use the delivery system for cosmetic and/or medical use obtainable by the process of the invention can be applied or implanted directly. Otherwise it can be reconstituted with water or other adequate solutions before application.

Another advantage of delivery system for cosmetic and/or medical use obtainable by the process of the invention is their fast release of some active ingredients, for example proteins. The delivery system for cosmetic and/or medical use obtainable by the process of the invention forms a hydrogel initially after contact with aqueous medium and releases a high active ingredient concentration into the surrounding aqueous medium at a controlled rate. Because of the locally applied and released active ingredients the achieved active ingredient concentration is much higher than a concentration achieved after release of active ingredients, which were incorporated into the xerogel (Fig. 1 A and B). Therefore lower amounts of applied active ingredients are necessary and material costs can be reduced.

Another important aspect of the presented invention is the possibility of controlled release kinetics. Active ingredients, which are applied on that side of a xerogel or film, which gets into contact with the release medium, are released very fast, whereas active ingredients, which are applied on the opposite side of the xerogel or film, are released at a controlled slow rate. Active ingredients on the opposite side initially have to diffuse through the whole xerogel or film, before they are released into the surrounding medium (Fig. 1 A and C). By combining these types of applied active ingredients (Fig. D), or by combining printed active ingredients applied according to the method of the invention with active ingredients incorporated into the xerogel or film the release kinetic can be varied and controlled.

In a preferred embodiment the invention relates to the use of a delivery system or a rehydrated delivery system or a composition as described above for cosmetic treatment.

In another preferred embodiment the invention relates to the use of a delivery system or a rehydrated delivery system or a composition as described above as medicament.

In another preferred embodiment the invention relates to the use of a delivery system or a rehydrated delivery system or a composition as described above for the manufacture of a medicament for treating wounds, skin diseases, ocular diseases and/or diseases of a mucosa. It also relates to a method of treating wounds, skin diseases, ocular diseases and/or diseases of a mucosa by administering a delivery system or a rehydrated delivery system or a composition as described above.

Although the products of the invention are particularly well suited for pharmaceutical and cosmetic use, they are not limited to that application and may also be designed for any industrial use, where defined application of stabilized compounds and materials on a xerogel or film and subsequent release with defined kinetics is warranted or useful.

Accordingly, the present invention, namely the methods and compositions of the present inventions can be applied more broadly, encompassing applicability not only to active ingredients but also to any chemical compound or material that is desired to be applied, administered or used.

These methods are only meant to be illustrative and are not limiting. Consequently they can be subjected to any modification desired.

The delivery systems obtainable according to the inventions as well as rehydrated systems and compositions comprising such delivery systems may be employed both in animals and in humans.

The versatility of the present invention is illustrated, but not limited, by the following examples.

All references cited are herewith incorporated herein in their entirety.

### Summary of Figures and Drawings

**Fig. 1:** Schematic illustration of release ways of active ingredients from delivery systems obtainable by the method of the invention, which were placed with the printed side face to face with the release side (A) or visa versa (C) or a combination of both (D) and of proteins incorporated into a xerogel or film (B).
**Fig. 2:** 10 µl placebo-colour-formulation was printed (25 nl droplets) on hydroxyethylcellulose xerogel. Photos were taken under a microscope (magnification factor 10x) of parts of the printed patterns.
**Fig. 3:** 20 µl placebo-colour-formulation was printed on ethylcellulose xerogels. The dry product (left picture) was rehydrated with pure water and the dissolution of the colour and the rehydration of the xerogel was monitored optically after 3 h (right picture). Still a clearly defined pattern is visible.
**Fig. 4:** Release of rh Erythropoietin monomer from different products. A: printed xerogel placed face to face with the release side to the membrane, B: printed xerogel placed visa versa, C: xerogel with incorporated protein, D: protein solution placed into the release cell.
**Fig. 5:** Release of rh G-CSF monomer from different products. A: printed xerogel placed face to face with the release side to the membrane, C: xerogel with incorporated protein, D: protein solution placed into the release cell.

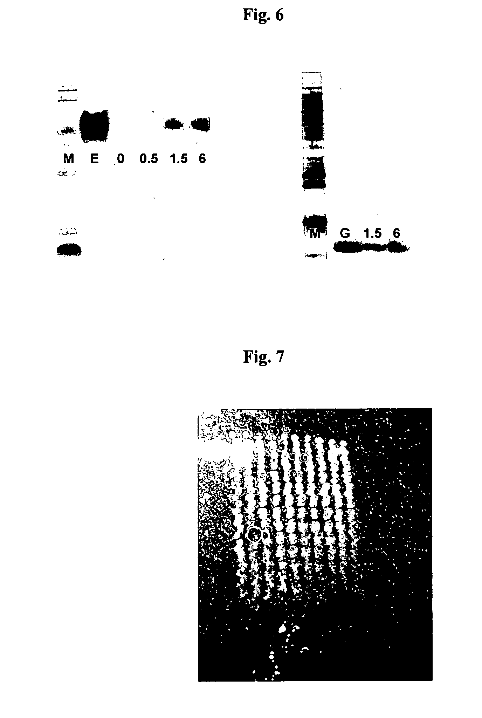

**Fig. 6:** SDS-PAGE gels of samples with Epo (left) or G-SCF (right) released from xerogels, which were prepared according to the presented method. M: marker, E: Epo formulation, G: G-CSF formulation, 0: drug free HEC, 0.5 to 6: after 0.5 to 6 hours of release.
**Fig. 7:** Appearance of a printed film product, created by application of 7,5 µl placebo-colour-formulation (dispensed into 25 nl droplets) on a hydroxyethylcellulose film.

### Examples

The following examples illustrate the invention and are non-limiting embodiments of the invention claimed.

### Example 1

In order to verify the controlled optical appearance of products prepared by the presented method a placebo-colour-formulation was printed on different xerogels.

Composition of the solution to be printed:
sucrose 5 %
phenylalanine 2 %
polysorbate 80 0,01 %
carboxyfluorescein q.s.
aqua purificata 20 µl

Xerogels were prepared from hydrogels containing 2% gelling agent (methyl-cellulose, ethylcellulose, polyacrylate or hydroxethylcellulose) by freeze-drying. The obtained xerogels had a diameter of 2 cm and a height of 3 mm.

10 µl placebo-colour-formulation was dispensed into 400 droplets of 25 nl. The droplets were placed 1 mm apart from each other on a pattern of 20 x 10 droplets two times in succession. The printed product was dried in vacuum at 20°C over night (pressure was reduced in five steps within one hour to 0,001 mbar and was held there for 14 hours).

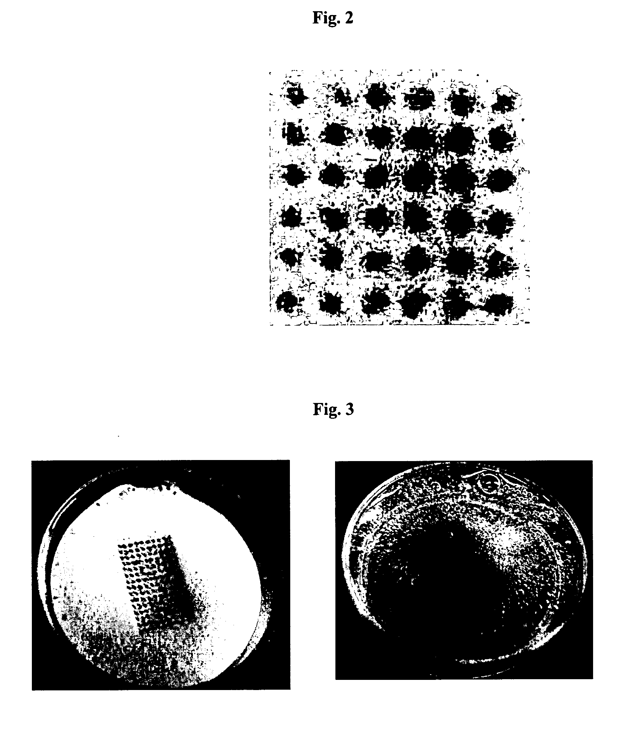

All products showed defined patterns (Fig. 2 shows the printed hydroxyethylcellulose-xerogel, products created from other xerogel materials were equivalent and are not presented). The applied droplets did not rehydrate the xerogels, but formed small, clearly defined, dry dots.

### Example 2

Additionally the optical appearance of products, which differ in the printed droplet sizes, was evaluated.

20 µl placebo-colour-formulation (from example 1) was printed on a hydroxyethylcellulose xerogel (prepared according to example 1) in the following pattern: 20 x 10 droplets, 1 mm apart from each other. Droplets of 25 nl were printed four times in succession in the same pattern, droplets of 50 nl were printed two times in succession. The printed products were dried in vacuum at 20°C over night (according to example 1).

Both products showed defined patterns. The applied droplets did not rehydrate the xerogels, but formed small dry dots. No difference in applied dry dot size was visible independent whether 25 nl large droplets were applied four times in succession on the same spot or whether larger droplets (50 nl) were printed twice on the same spot.

### Example 3

The residual moisture of active ingredient free and printed xerogels (printed and vacuum dried according to the presented method) was evaluated.

20 µl of placebo formulation (according to example 1, but without colour) was printed four times in succession as 25 nl droplets (according to example 2) on methylcellulose, polyacrylate or hydroxyethylcellulose xerogels (prepared according to example 1). The products were dried in vacuum (according to example 1).

Both active ingredient free and printed xerogels showed low residual moistures between 0,5% and 1,2% (m/m) independent on the used xerogel material (Tabel 1). No significant increase in residual moisture in printed products due to the printing and drying step was detected.

The residual moisture was detected by coulometric Karl-Fischer-titration (KF 373, Metrohm GmbH&Co, D-Filderstadt). Sample preparation was done under nitrogen in a glove box.

Table 1 below shows the residual moisture (%) of active ingredient free and printed xerogels, prepared according to the presented method.

**Tabel 1**

| **material** | **residual moisture (%)** | |
|---|---|---|
| | **active ingredient free xerogel** | **printed xerogel** |
| polyacrylate | 1,2 | 0,9 |
| methylcellulose | 0,9 | 0,5 |
| ethylcellulose | 0,7 | 0,9 |

### Example 4

For illustration of the rehydration process, water was added to dry placebo-colour products and the optically appearance of the samples (dissolution and distribution of the colour and rehydration of the xerogel) was monitored.

20 µl colour-placebo-formulation (according to example 1) was printed in 25 nl droplets four times in succession (according to example 2) on ethylcellulose and hydroxyethylcellulose xerogels (prepared according to example 1). The products were dried in vacuum (according to example 1). Pure water in such a quantity as it was present in the hydrogel before preparation of the xerogel was added and the optical appearance of the samples was monitored.

Rehydration and gelling of the samples was very fast. The distribution of the colour within the xerogel/hydrogel and the surrounding solution was monitored optically. A localized dissolution of the applied colour dots was achieved on ethylcellulose (Fig. 3) and hydroxyethylcellulose (data not shown) xerogels. The applied defined patterns on these materials were clearly visible for several hours (in Fig. 3 after 3 hours). Throughout the contact with water the surface of the xerogels was rehydrated very fast, leading to a gelled layer, which entrapped the applied colour dots. Even after 15 minutes of rehydration single, clearly defined colour dots were visible on the hydroxyethylcellulose xerogel. Later on the entrapped colour diffused slowly into the surrounding hydrogel and formed a homogenous hydrogel.

### Example 5

This example illustrates the fast and controlled release kinetic of products prepared by the presented method. The following formulation (pH 7,4) was printed on hydroxyethylcellulose xerogels.

Composition of the solution to be printed:

| | |
|---|---|
| sucrose | 0,001 g |
| phenylalanine | 0,0004 g |
| polysorbate 80 | 0,002 µl |
| rh Erythropoietin (2,4 mg/ml) | 20 µl |

Xerogels were prepared from hydrogels containing 2% gelling agent by freeze-drying. The obtained xerogels had a diameter of 2 cm and a height of 3 mm. 20 µl formulation was dispensed into 800 25 nl droplets. The droplets were placed 1 mm apart from each other on a pattern of 20 x 10 droplets four times in succession. The printed product was dried in vacuum at 20°C over night (pressure was reduced in five steps within one hour to 0,001 mbar and was held there for 14 hours).

The release kinetic of the applied rh Erythropoietin (Epo) monomer from rehydrated xerogel, through a cellulose acetate membrane into pure water (release medium) was monitored in a release cell for several hours at room temperature. Printed xerogels were placed into the cell once with the applied protein side face to face with the membrane (experiment A) and once in the opposite direction (experiment B). At the beginning of the experiment the dry products were rehydrated with pure water in such quantity as was present in the hydrogel before freeze-drying. The concentration of the protein monomer in the release medium was detected by SE-Chromatography after defined times.

The release kinetics are presented in Fig. 4. Reproducible release kinetics were achieved, whereby the release rate could be influenced by the orientation of the applied proteins on the xerogel relative to the release medium. If the printed xerogel side was placed near to the membrane a fast release into the release medium was achieved, whereby proteins from xerogels, that were placed visa versa into the release cell, had to diffuse through the whole rehydrated xerogel and were release much slower (Fig. 1).

### Example 6

The release of rh Epo from products prepared by the presented method (according to example 5) was compared with the dissolution of rh Epo, which was incorporated into a xerogel or which was in solution.

The release of protein monomer from the different products, through a cellulose acetate membrane into pure water was monitored in a release cell for several hours (according to example 5). Printed xerogels (experiment A and B, according to example 5) and a xerogel with incorporated protein (experiment C) were placed into the release cell and were reconstituted with pure water in such quantity as was present in the hydrogel before freeze-drying. For experiment D a protein solution with a concentration, that was calculated from the assumption, that all applied protein droplets are dissolved not in the whole xerogel volume, but in a 0,1 mm thin layer. The release kinetics of the different products were detected and are presented in Fig. 4.

The different release rates were caused by the difference in diffusion distance into the release medium and concentration of the applied proteins. Printed xerogels, which were placed with the release side face to face with the membrane (A), had the shortest diffusion distance (Fig. 1 A). After rehydration the applied droplets on the xerogel locally formed a very high concentrated protein solution next to the membrane, which preferable diffused away from the highly viscous gel through the membrane into the release medium. Therefore sample A showed the fastest release rate, even faster than the diffusion rate from the concentrated solution of sample D. The proteins in sample B and C had to diffuse out of or through the hydrogel and therefore showed slower release rates. Hereby was the diffusion distance from the printed xerogel, which was placed away from the release medium, the longest.

The release from all products was diffusion controlled. In the Higuchi-plot all data formed straight lines.

### Example 7

This example shows that it is also possible to print quite sensitive proteins, for example rh G-CSF (Granulocyte Colony Stimulating Factor), on xerogels with the presented method and release them with a fast kinetic. The products were prepared according to example 5.

Composition of the solution (pH 4) to be printed:

| | |
|---|---|
| sucrose | 0,001 g |
| phenylalanine | 0,0004 g |
| polysorbate 80 | 0,002 µl |
| rh G-CSF (4,2 mg/ml) | 20 µl |

The release kinetic of the applied rh G-CSF monomer from rehydrated xerogel, through a cellulose acetate membrane into pure water was monitored in a release cell (according to experiment 5) for several hours at room temperature. Printed xerogels were placed into the cell with the applied protein side face to face with the membrane (experiment A). At the beginning of the experiment the dry products were rehydrated with pure water in such quantity as was present in the hydrogel before freeze-drying. The concentration of the protein monomer in the release medium was detected by SE-Chromatography after defined times.

The release kinetics are presented in Fig. 5 (A) Fast reproducible release kinetics were achieved.

### Example 8

The release of rh G-CSF from products prepared by the presented method (according to example 7) was compared with the dissolution of rh G-CSF, which was incorporated into a xerogel or which was in solution.

The release of protein monomer from the different products, through a cellulose acetate membrane into pure water was monitored in a release cell for several hours. Printed xerogels (experiment A, according to example 7) and a xerogel with incorporated protein (experiment C) were placed into the release cell. They were reconstituted with pure water in such quantity as was present in the hydrogel before freeze-drying. For experiment D a protein solution with a concentration, that was calculated from the assumption, that all applied protein droplets are dissolved not in the whole xerogel volume, but in a 0,1 mm thin layer. The release kinetics of the different products were detected (Fig. 5).

The different release rates were caused by the difference in diffusion distance into the release medium and concentration of the applied proteins. Printed xerogels, which were placed with the release side face to face with the membrane, had the shortest diffusion distance (A). After rehydration the applied droplets on the xerogel locally formed a very high concentrated protein solution next to the membrane. Therefore sample A showed the fastest release rate, even faster than the diffusion rate from the concentrated solution of sample D. The proteins in sample C had to diffuse out of the hydrogel and therefore showed slower release rates.

### Example 9

This example illustrates the stability of proteins released from products prepared by the presented method. The following formulations were printed on hydroxyethylcellulose xerogels.

Composition of the solution 1 (pH 7,4) to be printed:

| | |
|---|---|
| sucrose | 0,001 g |
| phenylalanine | 0,0004 g |
| polysorbate 80 | 0,002 µl |
| rh Erythropoietin (2,4 mg/ml) | 20 µl |

Composition of the solution 2 (pH 4,0) to be printed:

| | |
|---|---|
| sucrose | 0,001 g |
| phenylalanine | 0,0004 g |
| polysorbate 80 | 0,002 µl |
| rh G-CSF (4,2 mg/ml) | 20 µl |

Xerogels were prepared from hydrogels containing 2% gelling agent by freeze-drying. The obtained xerogels had a diameter of 2 cm and a height of 3 mm. 20 µl formulation was dispensed into 400 50 nl droplets. The droplets were placed 1 mm apart from each other on a pattern of 20 x 10 droplets two times in succession.

The release of protein from the different products through a cellulose acetate membrane into pure water was monitored in a release cell for several hours. The released solutions were analysed for protein stability by SDS-PAGE.

The SDS-PAGE gels showed only monomer bands, no dimers, higher multimers or aggregates were detected. The released samples contained only native protein.

### Example 10

Storage stability of proteins printed on PE-foil (as a model system for a dry film carrier) according to the presented method was monitored. Products were prepared according to example 9 on PE foils. The products were analysed directly after production and after storage for 9 months at 8°C and 25°C.

Residual moisture of all products (detected by Karl-Fischer-Titration) did not exceed 1% (stored in a freezer) or 2%, when stored at 25°C for 9 months. Protein stability (detected by SEC-HPLC) was very good directly after production and throughout storage. Less than 2% of dimers, multimers and aggregates were formed in the products stored at 8°C or 25°C. No higher aggregates (detected by SDS-PAGE) were formed.

The protein monomer content (%) in samples, prepared according to the presented method, throughout storage. SPT stands for an aqueous solution containing 5% sucrose, 2% phenylalanine and 0,01%Polysorbate 80. SPT stands for an aqueous solution containing 5% sucrose and 0,01 %Polysorbate 80 is shown in Table 2 below. Concentrations are given as (w/v).

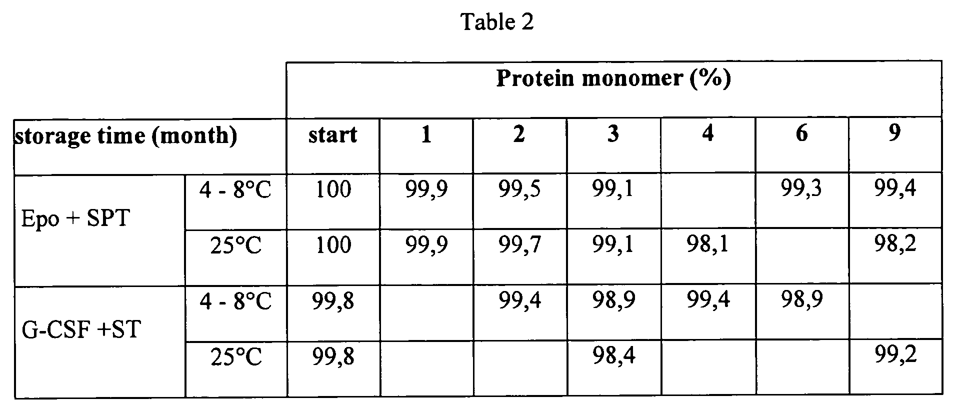

### Example 11

In order to verify the optical quality of printed film products prepared by the presented method a coloured formulation was printed on a dry film matrix.

Composition of the solution to be printed:

| | |
|---|---|
| sucrose | 5% |
| phenylalanine | 2 % |
| polysorbate 80 | 0.01 % |
| carboxyfluorescein | q.s. |
| aqua purificata | 20 µl |

Films were prepared from hydrogels (of a thickness of 1 mm) containing 5% gelling agent (hydroxethylcellulose) by air-drying. The obtained dry film matrixes were cut into pieces of 6 cm². 7,5 µl placebo-colour-formulation was dispensed into 300 droplets of 25 nl. The droplets were placed on a pattern of 15 x 10 droplets two times in succession, whereby the droplet rows were placed 1 mm apart from each other.

All products showed defined patterns. The applied droplets did not rehydrate or warp the films, but formed small, clearly defined, dry dots.

## Claims

1. Method of producing a delivery system for medical and/or cosmetic use comprising a carrier and at least one active ingredient **characterized by** following steps:
(i) preparing a liquid wherein at least one active ingredient is dissolved or dispersed
(ii) optionally sterilising the liquid
(iii) preparing and optionally sterilizing a dry xerogel or film carrier
(iv) applying microdroplets of the liquid according to steps (i) or, if applicable, (ii) to at least one surface area of a dry xerogel or film carrier obtained from step (iii)
(v) optionally repeating step (iv) at least one time, and
(vi) optionally repeating steps (i) to (v) with a liquid containing another active ingredient at least one time
(vii) vacuum-drying or freeze-drying the system obtained by above steps.

2. Method of producing a delivery system for medical and/or cosmetic use comprising a carrier and at least one active ingredient according to claim 1, **characterized by** following steps:
(i) preparing a liquid wherein at least one active ingredient is dissolved or dispersed
(ii) optionally sterilising the liquid
(iii) preparing and optionally sterilizing a dry xerogel or film carrier
(iv) applying microdroplets of the liquid according to steps (i) or, if applicable, (ii) to at least one surface area of a dry xerogel or film carrier obtained from step (iii)
(v) optionally repeating step (iv) at least one time, and
(vi) optionally repeating steps (i) to (v) with a liquid containing another active ingredient at least one time
(vii) vacuum-drying or freeze-drying the system obtained by above steps.

3. Method of producing a delivery system for medical and/or cosmetic use comprising a carrier and at least one active ingredient according to claim 1 or 2 **characterized by** following steps: (i) preparing a liquid wherein at least one active ingredient is dissolved or dispersed; (ii) sterilising the liquid; (iii) preparing and sterilizing a dry xerogel or film carrier; (iv) applying microdroplets of the liquid according to step (ii) to at least one surface area of a dry xerogel or film carrier obtained from step (iii); (v) optionally repeating step (iv) at least one time, and; (vi) optionally repeating steps (i) to (v) with a liquid containing another active ingredient at least one time; (vii) vacuum-drying or freeze-drying the system obtained by above steps.

4. Method according to any of claims 1 to 3, **characterized in that** the dry xerogel carrier is formed from a hydrogel by freeze-drying processes.

5. Method according to any of claims 1 to 3, **characterized in that** the dry film carrier is formed from a hydrogel by evaporative-drying processes, preferably air-drying, vacuum-drying or convective-drying.

6. Method according to any of claim 1 to 5, **characterized in that** the dry xerogel or film carrier contains one or more swellable, dissolvable or erodable polymers.

7. Method according to any of claim 1 to 6, **characterized in that** the gel-forming material of the dry xerogel or film carrier is selected from polysaccharides, like alginates, pectins, carrageenans or xanthan, starch and starch derivatives, gums like tragacanth or xanthan gum, collagen, gelatin, galactomannan and galactomannan derivatives, chitosan and chitosan derivatives, glycoproteins, proteoglycans, glucosaminoglycans, polyvinyl alcohol, polyvinylpyrrolidone, vinylpyrrolidone/vinyl acetate copolymers, high molecular weight polyethylene glycols and/or high molecular weight polypropylene glycols, polyoxyethylene / polyoxypropylene copolymers, polyvinyl alcohol, polyacrylates and/or polymethacrylates, tpolylactides, polyglycolides and polyaminoacids and/or cellulose derivatives.

8. Method according to claim 7, **characterized in that** the gel-forming material of the dry xerogel or film carrier is selected from cellulose derivatives, preferably methylcellulose, hydroxypropylcellulose, hydroxyethylcellulose, hydroxypropylmethylcellulose, sodium carboxymethylcellulose, ethylcellulose, cellulose acetate phthalate, hydroxypropylmethylcellulose phthalate, cellulose acetate succinate or ethylcellulose succinate or mixtures thereof.

9. Method according to any of claims 1 to 8, **characterized in that** the carrier contains one or more additional excipients like sugars, sugar alcohols, surfactants, amino acids, antioxidants, polyethylene glycols.

10. Method according to any of claims 1 to 9, **characterized in that** the carrier has at least two surfaces separated by edges.

11. Method according to any of claims 1 to 10, **characterized in that** the carrier has approximately the form of a cylinder, a sheet, a cube or a cuboid.

12. Method according to any of claims 1 to 11, **characterized in that** the microdroplets are applied to one or more surfaces, preferably one or two surfaces.

13. Method according to any of claims 1 to 12, **characterized in that** the microdroplets are applied in a way that the carrier essentially does not change its shape.

14. Method according to any of claims 1 to 13, **characterized in that** the microdroplets have a volume between about 0,05 nl and 10 µl, more preferably between about 0,5 nl and 200 nl, most preferably between about 10 nl and 100 nl.

15. Method according to any of claims 1 to 14, **characterized in that** the microdroplets in step (iv) of claims 1 to 2 are placed separately or with contact to each other or on top of each other, preferably separately.

16. Method according to any of claims 1 to 14, **characterized in that** the microdroplets are applied in a way that defined spots containing at least one active ingredient are obtained.

17. Method according to any of claims 1 to 3, **characterized in that** the microdroplets of step (vi), containing another active ingredient are applied separately or with contact to or on top of the microdroplets of the first round of step (iv), preferably separately from microdroplets of the first round of step (iv) of any of claims 1 to 3.

18. Method according to any of claims 1 to 3, **characterized in that** the microdroplets of step (vi), containing another active ingredient are applied to a different surface than the microdroplets applied in the first round of step (iv) any of claims 1 to 3.

19. Method according to any of claims 1 to 18, **characterized in that** the microdroplets are applied in the middle area of a carrier surface, leaving an active ingredient- free edging around said surface area.

20. Method according to claim 1 to 19, **characterized in that** the liquid of step (i) in claims 1 to 3 contains one or more excipients selected from sugars, sugar alcohols, surfactants, amino acids, buffers, lyoprotectants or antioxidants.

21. Method according to any of claims 1 to 20, **characterized in that** at least one active ingredient is a protein, peptide, RNA, DNA or another substance potentially unstable in a formulation.

22. Method according to any of claims 1 to 21, **characterized in that** at least one active ingredient is a substance, that promotes wound healing, preferably a wound healing factor, enzyme or proteinase inhibitor.

23. Delivery system for medical and/or cosmetic use comprising a carrier and at least one active ingredient, obtainable by a method according to any of the foregoing claims.

24. Method of rehydrating a delivery system according to claim 23 **characterized in that** the composition is brought into contact with an aqueous solution or water outside the patient to be treated.

25. Rehydrated delivery system obtainable by the method according to claim 24.

26. Rehydrated delivery system according to claim 25, wherein a fast release of at least one active ingredient is observed.

27. Rehydrated delivery system according to claim 25, wherein a slow, controlled release of the active ingredient or ingredients is observed.

28. Composition for cosmetic or medical application on skin or on skin wounds, comprising a delivery system according to claim 23 or a rehydrated delivery system according to claim 24 and an inert support, preferably selected from adhesive strip, adhesive wrap, bandage, gauze bandage, compress system.

29. Use of a delivery system according to claim 23 or a rehydrated delivery system according to claim 24 or a composition according to claim 28 for cosmetic treatment.

30. Use of a delivery system according to claim 23 or a rehydrated delivery system according to claim 24 or a composition according to claim 28 as medicament.

31. Use of a delivery system according to claim 23 for the manufacture of a medicament for treating wounds, skin diseases, ocular diseases and/or diseases of a mucosa.

32. Method according to any of claims 1 to 23 **characterized in that** the microdroplets are applied on one or more surfaces or surface areas of the carrier by means of printing or spotting, preferably by piezoelectric printers, more preferably by printers, which use a syringe pump and a high-speed micro-solenoid valve.

33. Method according to any of claims 1 to 23 or 32 **characterized in that** the carrier, on which the microdroplets are applied, is heated preferably to not more than 40°C, more preferably to not more than 30°C after step (iv) any of claims 1 to 3.

34. Method according to any of claims 1 to 23 or 32 to 33 **characterized in that** the system is dried in vacuum after step (iv) of any of claims 1 to 3 to lower the residual moisture preferably below 5%, more preferably below 2%, especially preferably below 1%.

35. Method according to any of claims 1 to 23 or 32 to 34 **characterized in that** a sterile liquid according to step (ii) of any of claims 1 to 3 containing at least one active ingredient is applied on a sterile carrier according to step (iii) of any of claims 1 to 3 under aseptic conditions, whereby a sterile delivery system is produced.

36. Method according to any of claims 1 to 23 or 32 to 35 **characterized in that** the sterile liquid according to step (ii) of any of claims 1 to 3 is produced by sterile filtration under aseptic conditions.

37. Method according to any of claims 1 to 23 or 32 to 36 **characterized in that** the sterile carrier according to step (iii) of claim 3 is obtained by sterilization of the hydrogel by hot vapour or radiation and drying.

38. Delivery system for medical and/or cosmetic use comprising a dry xerogel or film carrier and a pattern of dried microdroplets, containing one or more active ingredients.

39. Delivery system according to claim 38, wherein the pattern is regular.
